Europäisches Patentamt

European Patent Office

Office européen des brevets

(11) Publication number: **0 277 747**
**A2**

(12) # EUROPEAN PATENT APPLICATION

(21) Application number: **88300631.4**

(22) Date of filing: **26.01.88**

(51) Int. Cl.⁴: **C12P 7/64** , C12P 7/62 , C11C 1/04

(30) Priority: **27.01.87 JP 15109/87**

(43) Date of publication of application:
**10.08.88 Bulletin 88/32**

(84) Designated Contracting States:
**AT BE CH DE ES FR GB GR IT LI LU NL SE**

(71) Applicant: **Suntory Limited**
**1-40 Dojimahama 2-chome Kita-ku**
**Osaka-shi Osaka-fu 530(JP)**

(72) Inventor: **Shinmen, Yoshifumi**
**S-304, 8-1 Enmyojitoriimae Oyamazaki-cho**
**Otokuni-gun Kyoto(JP)**
Inventor: **Yamada, Hideaki**
**19-1, Matsugasakikinomoto-cho Sakyo-ku**
**Kyoto-shi Kyoto(JP)**
Inventor: **Shimizu, Sakayu**
**14, Nishinokyohakuraku-cho Nakagyo-ku**
**Kyoto-shi Kyoto(JP)**

(74) Representative: **Ford, Michael Frederick et al**
**MEWBURN ELLIS & CO. 2/3 Cursitor Street**
**London EC4A 1BQ(GB)**

(54) **Process for production of eicosapentaenoic acid from algae.**

(57) A process for the production of a lipid containing eicosapentaenoic acid comprising the steps of preparing algae belonging to the genus Corallina and containing eicosapentaenoic acid, and obtaining a lipid containing eicosapentaenoic acid from the algae; a process for the production of eicosapentaenoic acid wherein the eicosapentaenoic acid is isolated from the above-mentioned lipid; a process for the production of a lipid containing eicosapentaenoic acid comprising the steps of preparing algae belonging to the genus Corallina and capable of synthesizing eicosapentaenoic acid, preparing a product containing enzymes for synthesizing eicosapentaenoic acid from the algae, converting a precursor for eicosapentaenoic acid to eicosapentaenoic acid in the presence of the product containing enzymes for synthesizing eicosapentaenoic acid, and obtaining a lipid containing eicosapentaenoic acid; and a process for the production of eicosapentaenoic acid from the above-mentioned enzyme reaction.

EP 0 277 747 A2

# PROCESS FOR PRODUCTION OF EICOSAPENTAENOIC ACID FROM ALGAE

The present invention relates to a new process for the production of eicosapentaenoic acid from algae.

Processes for the production of eicosapentaenoic acid by extracting and purifying same from fish oils or certain algae are known, as is an enzymatic process for the production of eicosapentaenoic acid using eicosapentaenoic acid - synthesizing enzymes from algae belonging to the genus Prophyra (Japanese Unexamined Patent Application No. 61-31092). However, in addition to the target eicosapentaenoic acid, these process provide various kinds of contaminant unsaturated fatty acids, and therefore, it is difficult to obtain eicosapentaenoic acid having a high degree of purity.

Note, a process for the production of eicosapentaenoic acid using algae belonging to the genus Corallina is not known.

Accordingly, the present invention provides new processes for the production of eicosapentaenoic acid from algae belonging to the genus Corallina.

More particularly, the present invention provides a process for the production of a lipid containing eicosapentaenoic acid comprising the steps of:

preparing algae belonging to the genus Corallina and containing eicosapentaenoic acid; and

obtaining lipid containing eicosapentaenoic acid from the algae.

Also, the present invention provides a process for the production of eicosapentaenoic acid comprising the steps of:

preparing algae belonging to the genus Corallina and containing eicosapentaenoic acid;

obtaining lipid containing eicosapentaenoic acid from the algae; and

isolating eicosapentaenoic acid from the lipid.

The present invention further provides a process for the production of lipid containing eicosapentaenoic acid comprising the steps of:

preparing algae belonging to the genus Corallina and capable of synthesizing eicosapentaenoic acid;

preparing a product containing enzymes for synthesizing eicosapentaenoic acid, from the algae;

converting a precursor for eicosapentaenoic acid to eicosapentaenoic acid in the presence of the product containing enzymes for synthesizing eicosapentaenoic acid; and

obtaining a lipid containing eicosapentaenoic acid.

Moreover, the present invention provides a process for the production of eicosapentaenoic acid comprising the steps of:

preparing algae belonging to the genus Corallina and capable of synthesizing eicosapentaenoic acid;

preparing a product containing enzymes for synthesizing eicosapentaenoic acid, from the algae;

converting a precursor for eicosapentaenoic acid to eicosapentaenoic acid in the presence of the product· containing enzymes for synthesizing eicosapentaenoic acid; and

obtaining eicosapentaenoic acid.

Generally, algae contain about 0.2 to 3.0% by weight of lipid, relative to the total weight of dry algae. In the case of red algae belonging to the genus Corallina, eicosapentaenoic acid comprises 40 to 65% of the total fatty acid in the lipid, and the remaining fatty acids are mainly saturated or monounsaturated fatty acids, for example, palmitic acid, palmitoleic acid, oleic acid and the like. Therefore, algae belonging to the genus Corallina is superior to fish oils, which contain a large amount of other highly unsaturated fatty acids such as docosahexanoic acid, as a source of eicosapentaenoic acid from the viewpoint of an easy purification.

Moreover, since algae of the genus Corallina contain a series of enzymes necessary for the synthesis of highly unsaturated fatty acids, especially eicosapentaenoic acid, the enzyme system can be used to convert a precursor such as oleic acid, linolic acid, linolenic acid, or the like to eicosapentaenoic acid. Therefore, algae belonging to the genus Corallina are useful for the production of eicosapentaenoic acid in a highly purified form.

In the present invention, any algae belonging to the genus Corallina and capable of producing eicosapentaenoic acid can be used. Such producer algae include Corallina pilulifera and Corallina sessilis. These algae naturally grow in groups at the low tide mark in both inland sea and open sea waters, and therefore, are available as a natural resource. Moreover, these algae can be artificially cultured according to a conventional procedure for culturing algae.

According to an embodiment of the present invention, eicosapentaenoic acid can be obtained from algae obtained as a natural resource or artificially cultured. In this embodiment, eicosapentaenoic acid is extracted from algae with an organic solvent. Although intact algae can be subjected to extraction, preferably prior to extraction, algae is disrupted to accelerate the extraction. As a preferable embodiment, algae is homogenated in water or an organic solvent such as methanol, chloroform or the like using a homogenizer. Preferably, before the extraction, the algae or algae homogenate is dried. Drying is carried out by, for example, lyophilization or air-drying. The dried cells are treated with an organic solvent or a mixture thereof, preferably under a nitrogen stream, to extract a lipid containing target fatty acids. The organic solvent or mixture thereof is, for example, ethers such as ethyl ether, hydrocarbons such as hexane, alcohols such as methanol or ethanol, halo-hydrocarbons such as chloroform or dichloromethane, petroleum ether, as well as a mixture of chloroform, methanol and water, or a combination of methanol and petroleum ether alternately used. By distilling off the solvent, a lipid containing concentrated target fatty acid is obtained.

Alternatively, wet algae or algae homogenate can be subjected to extraction. In such a case, a water-miscible solvent such as methanol or ethanol, or a water-miscible solvent comprising the water-miscible solvent and water or other organic solvent is used. The extraction procedure is the same as described for dried cells.

The lipid thus obtained contains target fatty acids in the form of a lipid compound such as fat. Although the target fatty acid can be isolated in the form of a free acid, it is preferably isolated in the form of an ester with a lower alcohol, for example, as methyl ester. By converting the target fatty acid to such an ester, it is easily separated from other lipid components, and from other fatty acids originally present in algae such as palmitic acid, oleic acid, linoleic acid and the like, which are also esterified at the same time as the target fatty acid is esterified. To obtain methyl ester of the target fatty acid, for example, the lipid prepared as described above is treated with a 5 to 10% hydrochloric acid solution in absolute methanol or a 10 to 50% $BF_3$ solution in methanol for 1 to 24 hours at a room temperature.

The mixture thus obtained is extracted with an organic solvent such as hexane, ethyl ether or ethyl acetate, to recover methyl ester of the target fatty acid. Next, the extract is dried over anhydrous sodium sulfate, and the solvent is distilled under a reduced pressure to obtain a residue mainly comprising a fatty acid mixture. The mixture contains, in addition to the target compound, methyl palmitate, methyl stearate, methyl oleate and the like. From the mixture, methyl eicosapentaenoate is isolated by column chromatography, low temperature crystallization, a urea-adducting method, or a combination thereof.

The isolated methyl ester of a target fatty acid is then hydrolyzed with an alkali and extracted with an organic solvent such as ethyl ether, ethyl acetate, or the like to obtain the target fatty acid in a free form.

Alternatively, a target fatty acid can be obtained, without conversion to methyl ester, by alkalolysis with, for example, 5% sodium hydroxide at a room temperature for 2 to 3 hours, followed by extraction of the fatty acids from the alkalolysis product and isolation of the target fatty acid.

In another embodiment of the process of the present invention, eicosapentaenoic acid is produced by eicosapentaenoic acid - synthesizing enzymes from algae of the genus Corallina. In this process, a precursor for eicosapentaenoic acid is converted into eicosapentaenoic acid by the above mentioned enzymes. The enzyme can be in the form of intact algae, sliced algae, homogenate of algae, immobilized product of the homogenate, and the like. Algae can be homogenated by a conventional homogenizer in water or preferably in a buffer, such as a 0.05M phosphate buffer (pH 7.0). The homogenate can be immobilized according to a conventional procedure used to immobilize cells or enzymes, for example, the bound method, the cross linkage method, the entrapped method, the adsorbed method, and the like.

As a precursor for eicosapentaenoic acid, i.e., a substrate for the enzyme reaction, acetic acid, butylic acid, caproic acid, caprylic acid, capric acid, lauric acid, myristic acid, myristoleic acid, palmitic acid, palmitoleic acid, stearic acid, oleic acid, linoleic acid, linolenic acid, octadecatetraenic acid, arachidic acid, eicosenoic acid, eicosadienoic acid, eicosatrienoic acid, eicosatetraenoic acid, and the like can be used. These fatty acids are used in a free form or as a salt such as sodium, potassium or ammonium salt, or an ester such as methyl ester or ethyl ester or a combination thereof. Alternatively, the above-mentioned fatty acids can be used as a component of fats or oils, such as triglycerides. The substrate is preferably used in an amount of 0.001 mg to 1 g relative to 1 mg of dried algae as an enzyme source.

The reaction for a conversion of the above-mentioned precursor to the target eicosapentaenoic acid in the presence of the above-mentioned enzyme-containing preparation is carried out in an appropriate aqueous reaction medium. The reaction medium is, for example, water or a buffer such as a phosphate buffer, Tris-HCl buffer or the like.

If desirable or necessary, an emulsion promotor, for example, a surfactant such as Tween or Triton, or bile powder may be added to the reaction medium in an amount of 0.001% to 1%.

The reaction is carried out at a pH value of 3 to 10, preferably 5 to 8, at a temperature of 5 to 40°C, preferably 10 to 30°C, for 30 minutes to 10 days, preferably 10 to 48 hours. The reaction is carried out under a stationary condition, or preferably with agitation or shaking to accelerate contact of the precursor with the enzyme-containing preparations for conversion of the precursor to eicosapentaenoic acid.

The recovery of eicosapentaenoic acid from the reaction mixture can be carried out by substantially the same procedure as described for the isolation of eicosapentaenoic acid from algae per se.

Examples

The present invention will now be further illustrated by, but is by no means limited to, the following examples.

Example 1.

50 g (wet weight) of Corallina pilulifera obtained from Wakasa bay, Fukui, Japan, were thoroughly washed with water, and lyophilized to obtain 12.5 g of dried algae. The dried algae was extracted with a single phase mixed solvent consisting of chloroform/methanol/water, according to the Bligh and Dyer method. The extractant was then evaporated to obtain 95 mg of total lipid. The lipid was treated with a mixture of absolute methanol and hydrochloric acid (95:5) for three hours at 20°C to convert fatty acids contained in the lipid to corresponding methyl esters. A chromatographic analysis of the resulting fatty acid methyl esters showed a content of 6% methyl palmitate, 32% methyl palmitoleate, 2% methyl stearate, 4% methyl oleate, 1% methyl linoleate, 11% methyl arachidonate, 40% methyl eicosapentaenoate, and 4% others. This fatty acid methyl ester mixture was separated by column chromatography using octa decysilane with elution by acetonitrile solution to obtain a fraction containing essentially exclusively methyl eicosapentaenoate. The fraction was then evaporated to obtain 19 mg of purified methyl eicosapentaenoate. The methyl eicosapentaenoate product thus prepared was compared with a commercial standard sample of methyl eicosapentaenoate by gas chromatography, high performance liquid chromatography, Mass spectrum analysis, and NMR analysis. As a result, the data from the analyses of the samples were identical. The amount of methyl eicosapentaenoate before purification was 2 mg/g of dried algae, and that after purification was 1.5 mg/g of dried algae. Methyl eicosapentaenoate thus obtained could be hydrolyzed by a conventional procedure to obtain free eicosapentaenoic acid.

Example 2.

20 g (wet weight) of Corallina pilulifera, as described in Example 1 and which had been stored for six months at -80°C in a frozen form, was thawed. To this algae was added 50 ml of 0.05M phosphate buffer (pH 7.0), and the whole was homogenated by a homogenizer for 15 to 20 minutes with ice-cooling to disrupt algae cells. The resulting homogenate was used as an enzyme solution to prepare the following reaction mixture.

Composition of reaction mixture Enzyme solution     0.5 ml
Substrate     2 mg
0.6% Tween 20 aqueous solution     0.4 ml
Sea water     0.1 ml

As a substrate, sodium stearate, sodium oleate, sodium linolate, and sodium linolenate were separately used. Moreover, a control (1) which did not contain a substrate, and a control (2) wherein 0.5 ml of a 0.05M phosphate buffer was used in place of the enzyme solution, were prepared. Each reaction was carried out in a test tube (16.5 φ × 160 mm) at 28°C for three hours while shaking at 240 rpm. After the reaction, the reaction mixture was cooled to -20°C to terminate the reaction, and the reaction mixture was immediately lyophilized. The lyophilizate was treated with a mixture of absolute methanol and hydrochloric acid (95:5) at 20°C for five hours to convert fatty acids in the reaction mixture to methyl esters thereof, and extracted with 4 ml of ethyl ether to extract the resulting methyl esters. The extracts obtained from the above-mentioned reactions were then subjected to chromatography to determine the composition of the methyl esters of the produced fatty acids. The amount of each fatty acid produced during the present reaction was calculated by subtracting a total of the measurements of control (1) and control (2) from the measurements of corresponding fatty acids, from each reaction. Table 1 shows the amount of substrates consumption and the amounts produced of fatty acids, i.e., eicosapentaenoic acid (EPA), arachidonic acid (ARA), oleic acid (OLA), linoleic acid (LIA), and linolenic acid (LNA).

## Table 1

| Substrate (sodium salt) (µg) | Consumption of substrate (µg) | Production of fatty acid | | | | |
|---|---|---|---|---|---|---|
| | | EPA (µg) | ARA (µg) | OLA (µg) | LIA (µg) | LNA (µg) |
| Stearate | 170 | 1.6 | 0.1 | 0.2 | 0.1 | trace |
| Oleate | 136 | 45 | 4.5 | - | 0.9 | trace |
| Linolate | 111 | 64 | 5.8 | 0.1 | - | trace |
| Linolenate | 81 | 34 | 0.5 | 0.1 | 0.2 | - |

As seen from Table 1, the substrates tested were converted to mainly eicosapentaenoic acid.

Example 3.

Algae Corallina sessilis was obtained from the sea at Iwate, Japan, and cut with scissors to prepare slices of the algae, which were then used to prepare the following reaction mixture.

Composition of reaction mixture 0.05 M phosphate buffer (pH 7.0)      0.5 ml
Slices of algae      0.2 g (wet weight)
Substrates      2 mg
0.6% Tween 20 aqueous solution      0.4 ml
Sea water      0.1 ml

The reaction, the treatment of the reaction mixture, and the analysis of the reaction product were carried out according to substantially the same procedure as described in Example 2. The results are shown in Table 2.

5

## Table 2

| Substrate (sodium salt) (µg) | Consumption of substrate (µg) | Production of fatty acid | | | | |
|---|---|---|---|---|---|---|
| | | EPA (µg) | ARA (µg) | OLA (µg) | LIA (µg) | LNA (µg) |
| Stearate | 220 | 0.8 | trace | 0.1 | 0.1 | trace |
| Oleate | 165 | 47 | 6.1 | – | 0.7 | trace |
| Linolate | 122 | 56 | 7.2 | 0.1 | – | trace |
| Linolenate | 101 | 61 | 1.0 | 0.2 | 0.3 | – |

As seen from Table 2, the substrates used were converted to mainly eicosapentaenoic acid.

**Claims**

1. A process for production of a lipid containing eicosapentaenoic acid comprising the steps of:
preparing algae belonging to the genus Corallina and containing eicosapentaenoic acid; and
obtaining lipid containing eicosapentaenoic acid from the algae.
2. A process according to claim 1, wherein the algae is selected from the group consisting of Corallina pilulifera and Corallina sessilis.
3. A process for the production of eicosapentaenoic acid comprising the steps of:
preparing algae belonging to the genus Corallina and containing eicosapentaenoic acid;
obtaining a lipid containing eicosapentaenoic acid from the algae; and
isolating eicosapentaenoic acid from the lipid.
4. A process according to claim 3, wherein the algae is selected from the group consisting of Corallina pilulifera and Corallina sessilis.
5. A process for production of a lipid containing eicosapentaenoic acid comprising the steps of:
preparing algae belonging to the genus Corallina and capable of synthesizing eicosapentaenoic acid;
preparing a product containing enzymes for synthesizing eicosapentaenoic acid from the algae;
converting a precursor for eicosapentaenoic acid to eicosapentaenoic acid in the presence of the product containing enzymes for synthesizing eicosapentaenoic acid; and
obtaining a lipid containing eicosapentaenoic acid.
6. A process according to claim 5, wherein the algae is selected from the group consisting of Corallina pilulipera and Corallina sessilis.
7. A process for production of eicosapentaenoic acid comprising the steps of:
preparing algae belonging to the genus Corallina and capable of synthesizing eicosapentaenoic acid;
preparing a product containing enzymes for synthesizing eicosapentaenoic acid from the algae;
converting a precursor for eicosapentaenoic acid to eicosapentaenoic acid in the presence of the product containing enzymes for synthesizing eicosapentaenoic acid; and
obtaining eicosapentaenoic acid.
8. A process according to claim 7, wherein the algae is selected from the group consisting of Corallina pilulipera and Corallina sessilis.
9. A process according to any one of claims 5 to 8, wherein the product containing enzymes for synthesizing eicosapentaenoic acid is selected from the group consisting of intact algae, sliced algae, homogenated algae, and immobilized homogenate of algae.

10. A process according to any one of claims 5 to 9, wherein the precursor for eicosapentaenoic acid is selected from the group consisting of acetic acid, butyric acid, caproic acid, caprylic acid, capric acid, lauric acid, myristic acid, myristoleic acid, palmitic acid, palmitoleic acid, stearic acid, oleic acid, linoleic acid, linolenic acid, octadecatetraenoic acid, arachidic acid, eicosenoic acid, eicosadienoic acid, eicosatrienoic acid, and eicosatetraenoic acid.